# EUROPEAN PATENT APPLICATION

(11) **EP 1 754 517 A1**
(43) Date of publication of application: **21.02.2007**
(21) Application number: 06013725.4
(22) Date of filing: 03.07.2006
(51) Int. Cl.: A61Q 5/12, A61Q 5/10, A61K 8/898, A61K 8/893, A61K 8/891, D06M 15/643, C08L 83/04, C08L 83/08

(54) **A fiber and hair fiber conditioning treatment composition**

(30) Priority: 17.08.2005 EP 05017806
(71) Applicant: The Procter and Gamble Company, Cincinnati, Ohio 45202 (US)
(72) Inventor: Godfrey, Simon Paul, Uxbridge Middlesex UB8 1AR (GB)
(74) Representative: Kohol, Sonia

(57) **Abstract**

The present invention relates to a fiber or hair fiber conditioning treatment composition comprising a functionalised aminosilicone polymer and preferably in combination with a non functional aminosilicone polymer to provide improved conditioning without over conditioning negatives.

## Description

### BACKGROUND OF THE INVENTION

Oxidative dyeing, otherwise known as permanent coloring and hair bleaching lead to irreversible physico-chemical changes to the hair. Typically, during these processes, at least two components are mixed together prior to application to the hair. These usually comprise one component containing an oxidising agent, such as hydrogen peroxide, and a second component containing an alkaliser buffered at high pH, typically around 8.5 to 10.5 and optionally dyeing materials, such as oxidative dye precursors and couplers. After contacting with the hair, the mixture is left for a period of time suitable to allow the required color transformation to occur, after which the hair becomes more hydrophilic versus non-colored hair due to irreversible chemical changes. While not wishing to be bound by theory, this change in hair hydrophilicity appears to be due, among other things, to the oxidation of the keratin-keratin cystine amino acids within the hair creating more hydrophilic cysteic acid amino acid residues and to the removal by hydrolysis of nature's hydrophobic F-Layer. This coloring process is usually repeated regularly by consumers in order to maintain their desired hair color and color intensity and also to ensure that new hair growth has the same color as the older hair. As a consequence, the hair changes polarity from a relatively hydrophobic surface near the scalp where it could be experiencing its first color treatment to a progressively more polar substrate at the hair tips, which may have been subjected to multiple coloring treatments. A discussion of oxidation dyeing of hair can be found in "The Science of Hair Care" by Charles Zviak, Marcel Dekker, New York, 1986.

These irreversible physicochemical changes to the hair can also manifest themselves as increased roughness, brittleness and dryness leading to less manageable hair. The use of conditioners within the coloring process is known. Conditioning materials can be added to the colorant product, or alternatively these can be supplied within the colorant kit as a separate conditioner, and can thereby be applied to the hair either during the coloring event or after the colorant has been rinsed off. As described in EP 0 275 707, it is known to use aminosilicones for this purpose. However, it has also been established that, in the case of more polar hair, such as that obtained after successive oxidative colorings, aminosilicone deposition is greatly reduced and cannot provide the same level of benefit in hair condition as for non-oxidatively colored hair, especially when delivered within the harsh coloring environment. Without wishing to be bound by theory, the reason for this may be due to the existence of a surface energy incompatibility between the polar chemically damaged hair with the relatively non-polar aminosilicone, leading to poorer adhesion.

Improving deposition evenness between more and less damaged hair represents a major improvement area. This would enable sufficient deposition at the more polar/damaged tips for a noticeable conditioning benefit where it is needed most without over-depositing at the roots. Moreover, this technical challenge represents an even bigger issue across differing consumer populations. For instance, the varying damage levels found in different individuals can result in the same silicone generating acceptable deposition in some cases, but over-deposition, with the accompanying negative sensory implications, in others (e.g. first time colorers verses frequent colorers, brunettes verses bleached blondes etc). Hence, a silicone active that deposits across all hair types and damage levels is highly desirable.

Improving hair feel immediately after coloring is not the only desirable property of a colorant conditioner. After the coloring process, human hair becomes soiled due to its contact with the surrounding environment and from the sebum secreted from the scalp. This soiling of the hair causes it to have a dirty feel and unattractive appearance and necessitates shampooing with frequent regularity. Shampooing cleans the hair by removing excess soil and sebum, but can leave the hair in a wet, tangled, and generally unmanageable state. Once the hair dries, it is often left in a dry, rough, lustreless, or frizzy condition due to the removal of the hair's natural oils and other natural or deposited conditioning and moisturizing components. Hair can also be left with increased levels of static upon drying which can interfere with combing and result in a condition commonly referred to as "fly-away-hair". These conditions tend to be exaggerated on hair which has been previously oxidatively colored.

A variety of approaches have been developed to alleviate these post- shampoo problems. These approaches range from post-shampoo application of hair conditioners such as leave-on or rinse-off products, to hair conditioning shampoos which attempt to both cleanse and condition the hair from a single product. Hair conditioners are typically applied in a separate step following shampooing. The hair conditioners are either rinsed-off or left-on, depending upon the type of product used. Polydimethylsiloxanes (PDMS) are often employed as conditioning materials in both shampoo and conditioner applications to improve hair feel. However, it is known that, in the case of more hydrophilic hair obtained after oxidative coloring, PDMS deposition is greatly reduced, and cannot provide the same benefit in hair condition as for non-oxidatively colored hair.

Summarising some of the consumer needs discussed above, a dilemma faced by the present inventors was to produce a conditioner that is capable of depositing on hair of differing damage states, from undamaged, virgin hair, at the one extreme, to hair exposed to multiple oxidative dye treatments, at the other. Moreover there is an additional need for a conditioner, which can be used both immediately after hair coloring or bleaching and also as part of the consumer's regular hair grooming regime.

Attempts appear to have been made in the prior art to solve some of the problems discussed above. To be more specific, there has been a move away from using the highly hydrophobic PDMS-based silicones and towards using functionalized silicones, comprising functional groups such as amines, discussed above, and (among others) quaternary ammonium moieties. US 6,136,304, for example, discusses problems associated with conditioning compounds which are too easily rinsed from the hair. It also discusses application of conditioners to both virgin and damaged hair. The ethoxylated quaternary ammonium functionalized silicones proposed to achieve these aims, such as ABILQUAT 3272 and ABIL-QUAT 3270 (both having the CTFA designation of quaternium-80), produced by the Goldschmidt Chemical Corporation, Hopewell, Va, are so hydrophilic, however, that they are rapidly washed off during subsequent shampooings. In other words, they do not achieve sufficient durability to meet consumer needs. In terms of polarity, these silicones are at the other end of the spectrum from the PDMS-type materials, but they are similarly non-durable and therefore unsuitable. US 20030152542, US20030152541, US 20030152534, US 20030147842, US 20030147840, US 20030115685, US20030118537, US20030126692, US2000045098, US20030121108, US20030129155, US6824764, US6824765, US20030157049, US20030147827, US20030147841, US20030152543, US6846333, US20040163187 describe the use of several structural families of silicone used in conjunction with either: a conditioner, a thickener, an oxidizing agent, a reducing agent, a direct and / or oxidative dye or when used in a pre/post bleaching, pre or post coloring and during perming. The structural definition describes a variety of different degrees of functionalisation on the aminosilicone. However no understanding of the specific need to deposit onto colored hair, and the technical challenges this involves are described. Further, no understanding of the specific level of functionalisation, from the wide range described, to deliver silicone onto colored hair is demonstrated.

WO 98/43599, WO 03/075866, WO 99/29286, WO 99/49836, WO 99/34768, WO 03/092637, WO 03/096375 describe silicone containing conditioning compositions. These documents describe blends of an aminosilicone with various other silicones or hydrocarbon oils or aminosilicones with functionality higher than 1 MPC, where MPC refers to Mol PerCent and equates to the average number of functional groups substituted within 100 siloxane units. However, none of these describe the need or understanding of depositing silicone onto colored hair, or exemplify specific silicone structures which would deposit on to colored hair.

Alternatively the art (EP13558865, EP1356800, EP1356801, EP1356802 and EP 1357143) also describes the use of selected functionalised silicones, blends of selected silicones and combinations with additives to provide durable conditioning for colored hair which is maintained on the hair until the next coloring event. However; whilst such silicones provide improved hair feel, the consumer cannot use these conditioners as part of their regularly/daily hair care regime as these silicones would result in the hair becoming over conditioned causing the hair to feel greasy, stiff and hinder the ability to the style the hair.

With the above discussion in mind, the invention will ideally provide a fiber or hair fiber conditioning treatment composition comprising a functionalized silicone conditioning agent which can deposit on all types of hair, which occur in today's human population, from undamaged, virgin hair, at the one extreme, to hair exposed to multiple oxidative dye treatments, at the other.

Additionally, the invention will ideally provide a fiber or hair fiber conditioning treatment composition comprising a specific functionalized silicone conditioning agent which deposits over the whole length of a hair strand, including both lengths of uncolored scalp hair i.e. virgin hair and hair previously colored with an oxidative colorant.

Additionally, the present invention will ideally provide a fiber or hair fiber conditioning treatment composition comprising a specific functionalised aminosilicone polymer conditioning agent preferably in combination with a non functionalised silicone polymer for use on oxidation dyed or bleached hair, which can be used immediately after oxidative coloring or bleaching and as part of the consumer's post coloring treatment and also as conditioner used as part of their regular shampoo and conditioning routine without over conditioning negatives.

These and other features, aspects, and advantages of the present invention will become evident to those skilled in the art from a reading of the present disclosure.

### SUMMARY OF THE INVENTION

According to a first aspect of the invention, a fiber or hair conditioning treatment composition is presented, comprising a functionalized silicone according to the formula : 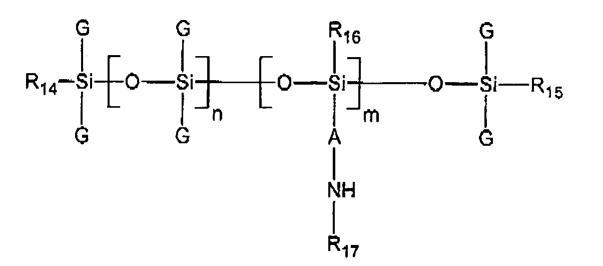 wherein, R₁₄, R₁₅, R₁₆, may be identical or different, and are selected from a hydroxyl radical, C1-C4 alkoxy radicals, methyl radical and mixtures thereof, A is selected from linear and branched C3-C8 alkyl radicals and mixtures thereof, R₁₇ is selected from H, phenyl, linear or branched C1-C4 alkyl radical, linear or branched (C1-C8)NH₂ and mixtures thereof, G is selected from H, phenyl, hydroxyl, C1-C8 alkyl and mixtures thereof, preferably methyl and wherein said functionalised aminosilicone polymer is a random type or block type and wherein m and n are both at least 1 or greater, the sum of (n+m) is greater than 50, and wherein the average ratio of m:n is 1:2000 to 1:200.

According to a second aspect of the present invention, the fiber or hair fiber conditioning treatment composition comprises the functionalized silicone as defined herein and further comprises a non functionalized silicone polymer, wherein the viscosity of said non functionalised silicone polymer is greater than 60000 cps.

As used herein, the term "fiber" includes strands of natural or synthetic materials. Non-limiting examples of natural materials are amino acid based materials, including protinaceous materials such as wool, human hair, including velus hair, and animal fur; cotton; cellulose and silk. Non-limiting examples of synthetic materials are polyester, nylon and rayon.

The fiber conditioning treatment composition according to the invention finds particular advantageous utility as a hair fiber treatment composition.

As used herein, the term "functionalized" silicone includes polydimethylsiloxanes (PDMS) in which at least one methyl group has been replaced by a different group, which contains a nitrogen atom. The term "functional silicone" is synonymous with the term "functionalized silicone". Similarly the term "non functionalised" silicone includes alkyl substituted silicones without any additional functionality, except terminal hydroxyl or alkoxy groups which remain due to the condensation polymerisation process.

The functional and non functionalised silicone components can be either miscible or non miscible with one another.

The term 'HLB value' is known to the skilled person working in this technical area; see for example Römpp Chemie Lexikon, Thieme Verlag, Stuttgart, 9th Edition, 1995 under "HLB-Wert".

### DETAILED DESCRIPTION OF THE INVENTION

All cited references are incorporated herein by reference in their entireties. All percentages given herein are by weight of total composition unless specifically stated otherwise. All ratios given herein are weight ratios unless specifically stated otherwise. All molecular weights given herein are weight average molecular weights, unless stated otherwise. All ratios of functional groups with silicone polymers are average ratios. Except where specific examples of actual measured values are presented, numerical values referred to herein should be considered to be qualified by the word "about".

In examining how to solve the above technical problems, the present inventors moved away from focusing exclusively on molecular properties and started also to consider the effects of altering the physical properties of silicones. That is because it was observed that silicone droplets tend to interact with strands of hair predominantly as fluids and not as individual molecules.
It has now been surprisingly found that functionalized aminosilicone polymers according to the formula below provides deposition on a range of hair types, without building up with re-use in a daily use context.

While not wishing to be bound by theory, it is believed that the presently claimed type of functional silicone provides an unexpected optimal performance from two different balancing performance vectors. These silicones are believed to have enough polarity to enable interaction with the more hydrophilic colored hair surface. However versus more functional aminosilicones, they do not have enough interaction with the colored hair to remain highly substantive during subsequent washing. Hence a sufficient proportion is removed during the next washing cycle to prevent "build-up" upon reapplication.

The functionalized silicones which may be incorporated into compositions according to the invention include silicones according to the formula: - 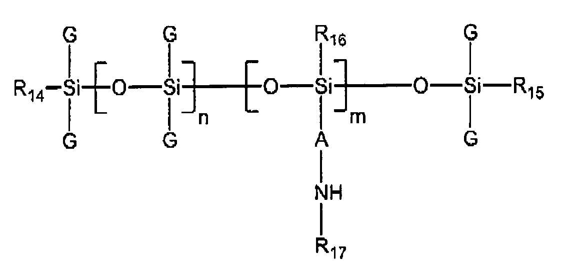 wherein, R₁₄, R₁₅, R₁₆, may be identical or different, and are selected from a hydroxyl radical, C1-C4 alkoxy radicals, methyl and mixtures thereof, A is selected from linear and branched C3-C8 alkyl radicals and mixtures thereof, R₁₇ is selected from H, phenyl, linear or branched C1-C4 alkyl radical, benzyl, linear or branched (C1-C8)NH₂ and mixtures thereof, G is selected from H, phenyl, hydroxyl, C1-C8 alkyl and mixtures thereof, preferably methyl, and wherein said functionalised aminosilicone polymer is a random type or block type and wherein m and n are both at least 1 or greater.

Preferably G is a methyl, R₁₄, R₁₅, R₁₆, which may be identical or different, are selected from a hydroxyl radical, C1-C4 alkoxy radicals and methyl, A is either (CH₂)₃ or (CH₂CHCH₃CH₂) and R₁₇ is either H or (CH₂)₂NH₂, and the average ratio m:n is 1:1500 to 1:200, more preferably 1:800 to 1:200, even more preferably 1:500 to 1:200 and most preferably 1:500 to 1:250 and the sum of m+n is in the range of 50 to 2000, preferably 100 to 1500, more preferably 300 to 1200, even more preferably 500 to 1000, most preferably 500 - 900 and even most preferably 550 - 850.

The following are non-limiting exemplary structures of the functionalized silicone according to the present invention.

### Example 1:

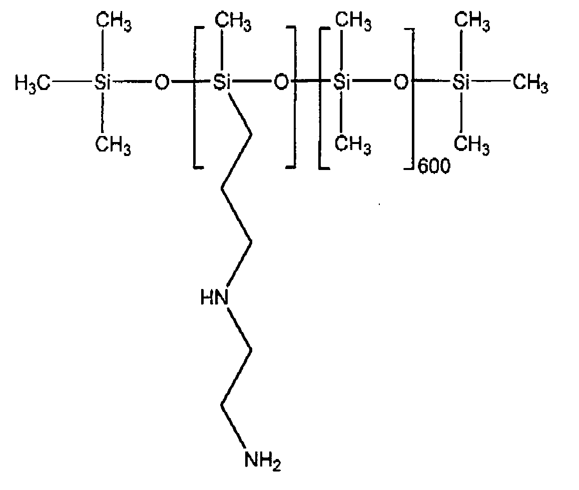

### Example 2:

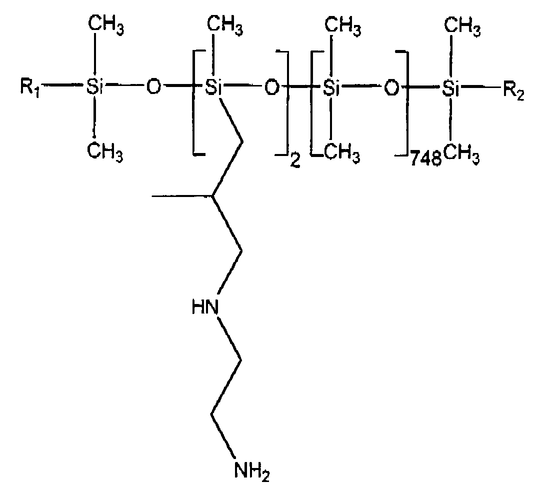

wherein R₁ and R₂ are a mixture of OH and OMe.

Preferably, the functionalized silicones of the present invention have a particle size of greater than 500 nm, preferably greater than 1 µm, more preferably greater than 2 µm. Particle size is determined according to the test method described hereinbelow.

The composition according to the present invention may comprise from 0.1 % to 10 % preferably 0.3 % to 9%, more preferably 0.5% to 8%, most preferably 1 % to 7.5% by weight of the functionalised aminosilicone polymer.

According to the present invention, the fiber conditioning treatment compositions preferably further comprises a non functionalized aminosilicone polymer. It has been surprisingly found that the benefits of the functionalized aminosilicone of the present invention can still be maintained if a portion of the functionalized aminosilicone is replaced by a non functionalized silicone. This has the advantage of reducing the cost associated with the compositions whilst still maintaining the benefits. Suitable non functionalized aminosilicone polymers are alkyl, phenyl or aryl substituted. Particularly preferred non functionalised silicones are selected from PDMS fluid and Gums with a viscosity greater than 60,000 cps.

The composition according to the present invention comprises from 0 % to 10 % preferably from 0.1 % to 9%, more preferably from 0.2 % to 8 %, most preferably from 0.5 % to 7 %, by weight of the non functionalised silicone polymer.

According to a further advantageous embodiment of the invention the blend of functionalized silicone and non functionalized silicone is present in an amount ranging from 0.1 to 20% by weight preferably from 0.5 to 10% by weight of the fiber conditioning treatment composition.

The functional and non functional silicone components may be blended either within the formula or pre-blended as a pre-mix prior to inclusion within the formula. Preferably, the functional and non functional silicone components are pre-blended as a pre-mix prior to inclusion.

According to the present invention the weight ratio of the functionalised aminosilicone polymer to the non functionalised silicone polymer, is from 5:95 to 95:5, more preferably 5:95 to 70:30, most preferably 10:90 to 50:50.

Preferably the non functionalised aminosilicone polymer has a particle size of greater than 500 nm, preferably greater than 1 µm, more preferably greater than 2 µm, as measured according to the method described hereinafter.

In a particularly preferred embodiment of the present invention the non functionalised aminosilicone has a viscosity greater than 60000cps as measured according to the method described hereinafter.

The silicones both functional and non functional can be prepared by methods known to those skilled in the art, via steps including known silicone polymerization reactions, for example equilibration or condensation.

The fiber conditioning treatment composition according to the present invention may include a cosmetically acceptable vehicle to act as a diluent, dispersant, or carrier for the silicone oil in the composition, so as to facilitate the distribution of the silicone oil when the composition is applied. The vehicle may be an aqueous emulsion, water, liquid or solid emollients, solvents, humectants, propellants, thickeners and powders.

Advantageously, the fiber conditioning treatment compositions according to the present invention may be in the form an emulsion with water as a primary component, although aqueous organic solvents may also be included. The emulsion is preferably an oil-in-oil-in-water (silicone-in-silicone-in-water) emulsion.

The aqueous continuous phase of the emulsion treatment compositions of the present invention may further comprise an emulsifier to facilitate the formation of the emulsion. Emulsifiers for use in the aqueous continuous phase of the present emulsion treatment compositions may include an anionic surfactant, cationic surfactant, amphoteric surfactant, water-soluble polymeric surfactant, water-soluble silicone-containing surfactant, nonionic surfactant having an HLB of greater than about 10, or a surfactant system capable of forming stabilizing liquid crystals around the silicone droplets. The nonionic surfactant preferably has an HLB of at least 12, and more preferably, an HLB value of at least about 15. Surfactants belonging to these classes are listed in McCutcheon's Emulsifiers and Detergents, North American and International Editions, MC Publishing Co., Glen Rock NJ, pages 235-246 (1993).

The emulsifier for the aqueous phase does not gel the aqueous phase. The emulsifier however may be capable of forming a stabilizing layer of lamellar liquid crystals around silicone droplets. This barrier film prevents coalescence between emulsion droplets. In this case, the surfactant system can be a single surfactant or a blend of surfactants. In some cases, a particular surfactant cannot form a liquid crystal structure alone, but can participate in the formation of liquid crystals in the presence of a second surfactant. Such a surfactant system forms a layer of lamellar liquid crystals around the silicone to provide a barrier between the silicone and the aqueous phase. This type of an emulsion is different from the conventional emulsions, which rely upon the orientation of the hydrophobic and hydrophilic components of a surfactant at an silicone-water interface. The formation of a layer of lamellar liquid crystals around the silicone can be detected by the presence of Maltese crosses viewed by optical microscopy through crossed polarizing plates or by freeze fracture electron microscopy.

Exemplary classes of surfactants capable of participating in the formation of a liquid crystal structure around the silicone droplets include, but are not limited to specific cationic surfactants, anionic surfactants, nonionic surfactants, quaternary ammonium surfactants, lipid surfactants and mixtures thereof.

Preferred surfactants for the formation of liquid crystals in the aqueous continuous phase are of the nonionic type and include C16-C22 fatty alcohols, and C16-C22 fatty alcohol ethoxylates with 1 to 30 ethylene oxide groups. Specific examples include cetearyl alcohol, cetyl alcohol, stearyl alcohol, arachidyl alcohol, oleyl alcohol, ceteareth ethoxylates with between 10 and 30 ethylene oxide groups, ceteth ethoxylates with between 10 to 30 ethylene oxide groups, steareth ethoxylates with between 10 and 30 ethoxylates, and combinations thereof. Preferably, C16-C22 fatty alcohols are used in combination with C16-C22 fatty alcohol ethoxylates at a ratio of between 10:1 to 0.5:1, more preferably between 6:1 and 1:1, and most preferably between 5:1 and 1.5:1.

Preferred cationic surfactants contain quaternary ammonium compounds of formula: [R₁₈R₁₉R₂₀R₂₁N]+X-, where R₁₈ is an alkyl or alkenyl group having from about 8 to 22 carbon atoms, R₁₉ and R₂₀ are both independently either an alkyl or alkenyl group having from about 8 to 22 carbon atoms or alkyl or hydroxyalkyl group having from about I to 4 carbon atoms, R₂₁ is an alkyl or hydroxyalkyl group having from about 1 to 4 carbon atoms, and X- is a salt forming anion (e.g. chloride, bromide, acetate, alkylsulfate). Specific examples include cetrimonium chloride, behentrimonium chloride and dicetyldimonium chloride.

Advantageously, in order to facilitate formation of liquid crystals, the surfactant system may also comprise amidoamines of the following general formula: R₂₂CONH(CH₂)m N (R₂₃)₂, wherein R₂₂ is a residue of C8-C24 fatty acids, R₂₃ is a C1-C4 alkyl, and m is an integer from 1 to 4. Preferred amidoamine useful in the present invention includes stearamidopropyldimethylamine, stearamidopropyldiethylamine, stearamidoethyl-diethylamine, stearamidoethyldimethylamine, palmitamidopropyldimethylamine, paimit-amidopropyldiethylamine, palmitamidoethyldiethylamine, palmitamido-ethyldimethylamine, behenamidopropyldimethylamine, behenamidopropyldiethylamine, behenamidoethyldiethylamine, behenamidoethyldimethylamine, arachidamidopropyldimethylamine, arachidamido-propyldiethylamine, arachidamidoethyldiethylamine, arachidamidoethyldimethylamine, and mixtures thereof; more preferably stearamido-propyldimethylamine, stearamidoethyldiethylamine, and mixtures thereof.

More advantageously, the amidoamines are partially quaternized with the acids selected from the group consisting of L-glutamic acid, lactic acid, hydrochloric acid, malic acid, succinic acid, acetic acid, fumaric acid, L-glutamicio acid hydrochloride, tartaric acid, and mixtures thereof; preferably L-glutamic acid, lactic acid, hydrochloric acid, and mixtures thereof. Preferably, the mole ratio of amidoamine to acid is from about 1:0.3 to about 1:1, more preferably from about 1:0.5 to about 1:0.95

The aqueous continuous phase should ideally comprise the emulsifier in an amount sufficient to stabilize the silicone. In one embodiment, the aqueous continuous phase comprises the emulsifier in an amount of from about 0.1% to about 15%, and more preferably from about 0.1 % to about 10%, based on the weight of the aqueous continuous phase.

The conditioning composition of the present invention may include optional benefit materials and cosmetic adjuncts, as long as the benefit materials or the adjuncts do not eliminate or substantially reduce the performance or shelf stability of the composition. The additional ingredients may include, for example dyes and coloring agents, fragrances; anionic, cationic, non-ionic, amphoteric or zwitterionic surfactants; buffers, masking fragrances, dispersing agents, stabilizers, cationic polymers, perfumes, non-ionic polymers, anionic polymers, complex coacervates, complex coacervate capsules, metal salts, lewis acids, buffering agents, particulate thickeners, polymeric thickeners, wax thickeners, oils, emollients, humectants, moisturizers, pearlescents, opacifiers, enzymes, suspending agents, antimicrobials, preservatives, proteins, herb and plant extracts, bleach, peroxide, polyols, silicones, antibodies, pH adjusting agents including pH buffers, viscosity modifiers, preservatives, viscosity enhancers, gelling agents, chelators, oxidising agents, reducing agents, UV filters, emulsifying agents, antioxidants, moisturizing and conditioning agents, and other common adjuvants well known to those skilled in the art.

The compositions of the present invention may also optionally further comprise additional conditioning agents such as silicones or aminosilicones not described within the aforementioned descriptions to further improve the conditioning performance.

The composition of the present invention may optionally further comprise at least about 0.01% of polymer as an additional thickener, rheology modifier, stabilizer and/or conditioning agent.

The polymer can be chosen, for example, from associative polymers. As used herein, the expression "associative polymer" means an amphiphilic polymer comprising both hydrophilic units and hydrophobic units, for example, at least one C8-C30 fatty chain and at least one hydrophilic unit. Representative associative polymers that may be used are associative polymers chosen from:
(i) nonionic amphiphilic polymers comprising at least one fatty chain and at least one hydrophilic unit; for example celluloses or hydroxyethylcelluloses modified with groups comprising at least one fatty chain, hydroxypropyl guars modified with groups comprising at least one fatty chain, polyether urethanes comprising at least one fatty chain, copolymers of vinylpyrrolidone and of fatty-chain hydrophobic monomers, copolymers of hydrophilic acrylates or methacrylates and of hydrophobic monomers comprising at least one fatty chain.
(ii) anionic amphiphilic polymers comprising at least one hydrophilic unit and at least one fatty-chain unit; these, for example, may be chosen from those comprising at least one fatty-chain allyl ether unit and at least one hydrophilic unit comprising an ethylenic unsaturated anionic monomeric unit, or from those comprising at least one hydrophilic unit of unsaturated olefinic carboxylic acid type, and at least one hydrophobic unit of the type such as a (C8-C30) alkyl ester or oxylakylenated (C8-C30) alkyl ester of an unsaturated carboxylic acid; anionic amphopilic polymers may be further cross-linked.
(iii) cationic amphiphilic polymers comprising at least one hydrophilic unit and at least one fatty-chain unit; these, for example, may be chosen from quaternized cellulose derivatives and polyacrylates comprising amino side groups.
(iv) amphoteric amphiphilic polymers comprising at least one hydrophilic unit and at least one fatty-chain unit; mention may be made, for example, of methacrylamidopropyltrimethylammonium chloride/acrylic acid/C10-C30 alkyl methacrylate copolymers, wherein the alkyl radical is, for example, a stearyl radical.
Further, the polymer can be chosen from crosslinked acrylic acid homopolymers, crosslinked copolymers of (meth)acrylic acid and of (C1-C6)alkyl acrylate or polysaccharides. The polymer may also serve as conditioning agents, as described below.

Particularly preferred are acrylates/stereath-20 methacrylate copolymer (Aculyn 22 supplied by Rohm and Haas) and acrylates copolymer (Aculyn 33 supplied by Rohm and Haas) and Xanthan gum, Carbopol, Rheozan and mixtures thereof. The polymer will generally be used at levels of from about 0.01% to about 20.0% by weight of the composition, preferably of from about 0.1 % to about 5%.

For use, the conditioning compositions according to an embodiment of the invention may be provided at a pH from about 3 to 11, preferably from 4 to 10.5.

The conditioning compositions according to the present invention may be provided in any suitable physical form, for example as low to moderate to high viscosity liquids, lotions, milks, mousses, dispersions, sprays, gels, foams, aerosols, and creams. These compositions may be produced by procedures well known to the skilled artisan.

The conditioning compositions of the present invention can be formulated as a fluid, lotion, fluid cream or cream having a viscosity from 500 to 100,000 cps or above. The compositions can be packaged in a suitable container to suit its viscosity and intended use by the consumer. For example, a lotion or fluid cream can be packaged in a bottle, a roll-ball applicator, a propellant-driven aerosol device, a container fitted with a pump suitable for hand or finger operation, or the like. When the composition is a cream, it can simply be stored in a non-deformable bottle or squeeze container, such as a tube or a lidded jar.

The hair fiber conditioner can be sold within a conditioning hair fiber coloring treatment kit. According to a further aspect of the invention, a hair conditioning fiber coloring treatment kit is provided comprising: (a) a first oxidative bleaching composition, (b) a coloring composition, and or (c) a second oxidative bleaching compostion wherein the hair fiber conditioning treatment composition as defined hereinabove is comprised within component (a) and/or within component (b), and or within component (c) and/or is provided as a separate component, which can be combined with the other components before use or used separately therefrom. Preferably, the hair fiber conditioning treatment composition is provided as a separate component. The composition according to the present application finds particular utility in hair bleaching, hair coloring and highlighting compositions especially oxidative hair colorants wherein the hair is subjected to a particularly aggressive environment.

Accordingly, the present invention may further comprise a hair coloring or bleaching composition comprising at least one source of an oxidizing agent and mixtures thereof. Preferred oxidizing agents for use herein are water-soluble peroxygen oxidizing agents. "Water-soluble" as defined herein means that in standard condition at least 0.1g, preferably 1g, more preferably 10g of said oxidizing agent can be dissolved in I liter of deionized water. The oxidizing agents are valuable for the initial solubilisation and decolorisation of the melanin (bleaching) and accelerate the oxidation of the oxidative dye precursors (oxidative dyeing) in the hair shaft. Preferred water-soluble oxidizing agents are inorganic peroxygen materials capable of yielding hydrogen peroxide in an aqueous solution, and include hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulphates and the like, which may be incorporated as monohydrates, tetrahydrates etc., and alkyl and aryl peroxides, and or peroxidases. The oxidizing agents may be provided in aqueous solution or as a powder which is dissolved prior to use. Preferred for use in the compositions according to the present invention are hydrogen peroxide, percarbonate, persulphates and combinations thereof. According to the present invention the hair coloring or bleaching compositions comprise from about 0.1% to about 15% by weight, preferably from about 1% to about 10% by weight, and most preferably from about 2% to about 7% by weight of an oxidizing agent.

Another preferred oxidizing agent for use herein is a source of peroxymonocarbonate ions. Preferably such a source is formed in situ from a source of hydrogen peroxide and a hydrogen carbonate ion source. Such an oxidizing agent has been found to be particularly effective at a pH of up to and including 9.5, preferably from 7.5 to 9.5 more preferably from 8.4 to 9.5 and most preferably at about pH 9. Moreover, this system is also particularly effective in combination with a source of ammonia or ammonium ions. It has been found that this oxidizing agent can deliver improvements to the desired hair color results particularly with regard to the delivery of high lift, whilst considerably reducing the odour, skin and scalp irritation and damage to the hair fibers.

Suitable sources for use herein include sodium, potassium, guanidine, arginine, lithium, calcium, magnesium, barium, ammonium salts of carbonate, carbamate and hydrocarbonate ions and mixtures thereof such as sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate and mixtures thereof. Percarbonate salts may also be utilized to provide both the source of carbonate ions and oxidizing agent. Preferred sources of carbonate ions, carbamate and hydrocarbonate ions are sodium hydrogen carbonate, potassium hydrogen carbonate, ammonium carbamate, and mixtures thereof.

The hair coloring or bleaching compositions comprise from about 0.1 % to about 15% by weight, preferably from about 1% to about 10% by weight, and most preferably from about 1% to about 8% by weight of a hydrogencarbonate ion and from about 0.1% to about 10% by weight, preferably from about 1% to about 7% by weight, and most preferably from about 2% to about 5% by weight of a source of hydrogen peroxide.

The hair coloring or bleaching compositions of the present invention may further comprise additional ingredients which include, but are not limited to; alkalising agents, surfactants, hair dyeing agents such as oxidative dye precursors, non-oxidative preformed dyes, thickeners and / or rheology modifiers, opacifiers such as mica, solvents, enzymes, surfactants, conditioning agents, carriers, antioxidants, stabilizers, chelants, perming actives, perfume, reducing agents, hair swelling agents and/or polymers. Some of these additional components are detailed hereafter.

According to the present invention the hair coloring or bleaching composition may optionally comprise at least one source of alkalizing agent, preferably a source of ammonium ions and or ammonia. Particularly, preferred alkalizing agents are those which provide a source of ammonium ions. Preferred sources include ammonium chloride, ammonium sulphate, ammonium nitrate, ammonium phosphate, ammonium acetate, ammonium carbonate, ammonium hydrogen carbonate, ammonium carbamate, ammonium hydroxide, percarbonate salts, ammonia and mixtures thereof. Particularly preferred are ammonium carbonate, ammonium hydrogen carbonate, ammonium carbamate, ammonia and mixtures thereof. The compositions of the present invention may comprise from about 0.1% to about 10% by weight, preferably from about 0.5% to about 5%, most preferably from about 1% to about 3% of an alkalizing agent, preferably ammonium ions. Preferably, if present, the ammonium ions and carbonate ions are present in the composition at a weight ratio of from 3:1 to 1:10, preferably 2:1 to 1:5.

Preferably, the hair coloring compositions have a pH of from about 11 to about 7, preferably from about 9.5 to about 7.5, more preferably from about 9.5 to about 8.4 and most preferably from about 9.4 to about 8.5 and even more preferably about pH 9.0. The pH of the compositions can be determined by using either a Mettler Toledo MP220 or a MP225 pH equipment, fitted with a standard laboratory pH electrode. The equipment is calibrated before each use using standard calibration buffers and using standard calibration procedure.

Preferably, hair coloring compositions comprise but are not limited to oxidative dyeing compositions. Such compositions comprise oxidative hair dye precursors also known as primary intermediates and couplers that will deliver a variety of hair colors to the hair. These small molecules are activated by the oxidizing agent and react with further molecules to form a larger colored complex in the hair shaft. The precursors can be used alone or in combination with other precursors, and one or more can be used in combination with one or more couplers. Couplers (also known as color modifiers or secondary intermediates) are generally colorless molecules that can form colors in the presence of activated precursors, and are used with other precursors or couplers to generate specific color effects or to stabilize the color. The choice of precursors and couplers will be determined by the color, shade and intensity of coloration that is desired. The precursors and couplers can be used herein, singly or in combination, to provide dyes having a variety of shades ranging from ash blonde to black. These compounds are well known in the art, and include aromatic diamines, aminophenols, aromaticdiols and their derivatives (a representative but not exhaustive list of oxidation dye precursor can be found in Sagarin, "Cosmetic Science and Technology", "Interscience, Special Edn. Vol. 2 pages 308 to 310).

The hair coloring compositions may also include non oxidative hair dyes. i.e. direct dyes which may be used alone or in combination with the above described oxidative dyes. Suitable direct dyes include azo or anthraquinone dyes and nitro derivatives of the benzene series and or melanin precursors and mixtures thereof. Such direct dyes are particularly useful to deliver shade modification or highlights. Particularly preferred are Basic Red 51, Basic Orange 31, Basic Yellow 87 and mixtures thereof.

The hair coloring compositions will generally comprise from about 0.001% to about 10% of dyes. For example compositions providing low intensity dyeing such as natural blonde to light brown hair shades generally comprise from about 0.001% to about 5%, preferably from about 0.1% to about 2%, more preferably from about 0.2% to about 1% by weight of dyeing composition of precursors and couplers. Darker shades such as browns and black typically comprise from 0.001% to about 10% by weight, preferably from about 0.05% to about 7% by weight, more preferably from about 1% to about 5% of precursors and couplers.

According to the present invention the hair coloring or bleaching compositions may optionally comprise chelants. Chelants are well known in the art and refer to a molecule or a mixture of different molecules each capable of forming a chelate with a metal ion. Chelants may be incorporated into the hair coloring or bleaching compositions as stabilizers and or preservatives. In addition, it has also been found that chelants provide hair fiber damage benefits and thus they may be utilized in order to further improve the hair damage profile of the present invention. Chelants are well known in the art and a non-exhaustive list thereof can be found in AE Martell & RM Smith, Critical Stability Constants, Vol. 1, Plenum Press, New York & London (1974) and AE Martell & RD Hancock, Metal Complexes in Aqueous Solution, Plenum Press, New York & London (1996) both incorporated herein by reference. Examples of chelants suitable for use herein include EDDS (ethylenediaminedisuccinic acid), carboxylic acids (in particular aminocarboxylic acids), phosphonic acids (in particular aminophosphonic acids) and polyphosphoric acids (in particular linear polyphosphoric acids), their salts and derivatives.

The hair coloring or bleaching compositions may further optionally comprise a solvent system. Suitable solvents for use in the solvent system herein include, but are not limited to, amides, esters, ethers, ketones, cyclic amides, cyclic esters, cyclic ketones, cyclic ethers, water and mixtures thereof. Nonlimiting examples of such solvents include ethyl formate, dimethyl isosorbide, acetylacetone, 2-butanone, acetone, methyl acetate, ethyl acetate, propyl acetate, ethoxyethanol, dipropylene glycol monomethyl ether, butyl lactate, t-butyl alcohol, phenyl acetate, 2-propoxyethanol, isopropoxyethanol, methoxypropanol, isopropyl lactate, hexyl alcohol, butoxyethanol, tripropylene glycol (PPG-3), triacetin, methoxyethanol, isopropyl alcohol, PEG-8, methyl lactate, PEG-6, PEG-5, PEG-4, N-methylpyrrolidone, propyl alcohol, dipropylene glycol (PPG-2), acetonitrile, phenoxyethanol, triethylene glycol, hexylene glycol, ethyl alcohol, γ-butyrolactone, butylene glycol, propylene carbonate, dimethyl sulfoxide, diethylene glycol, ethoxydiglycol, propylene glycol, pyrrolidone, pyrrolidone-2, methyl alcohol, ethylene carbonate, ethylene glycol, acetamide, glycerin, butyl carbitol, 1,3-dioxolane, dimethoxymethane, 1,2-hexanediol, dipropylene glycol butyl ether, dipropylene glycol t-butyl ether, propionaldehyde, diethoxymethane and glycerol formaldehyde.

Preferred solvents for use in the solvent system herein include lower alkanols, C2-C6 polyols, glycol mono-lower alkyl ethers, diglycol mono-lower-alkyl ethers, and N-lower alkylpyrrolidones. The term "lower" refers to the number of carbon atoms being 3 or less. Specific examples include lower alcanols such as Ethanol, Isopropyl alcohol, lower polyols such as ethylene glycol, propyleneglycol, 1,3-butanediol, diethyleneglycol, glycerine; glycol monoethers such as 2-methoxyethanol and 2-ethoxyethanol; diglycol mono-lower alkyl ethers such as methoxydiglycol, ethoxydiglycol and N-lower-alkylpyrrolidones such as N-methylpyrrolidone and N-ethylpyrrolidone.

The hair coloring or bleaching compositions may also comprise a source of radical scavenger. As used herein the term radical scavenger refers to a species that can react with a radical, preferably carbonate radical to convert the radical by a series of fast reactions to a less reactive species. The radical scavenger is also preferably selected such that it is not an identical species as the alkalizing agent and in present in an amount sufficient to reduce damage to hair during the colouring process. The compositions of the present invention preferably comprise from about 0.1% to about 10% by weight, preferably from about 1% to about 7% by weight of radical scavenger.

Preferred radical scavengers are selected from the classes of alkanolamines, amino sugars, amino acids, esters of amino acids and mixtures thereof. Particularly preferred compounds are: monoethanolamine, 3-amino-1-propanol, 4-amino-1-butanol,5-amino-1-pentanol, 1-amino-2-propanol, 1-amino-2-butanol, 1-amino-2-pentanol, 1-amino-3-pentanol, 1-amino-4-pentanol, 3-amino-2-methylpropan-1-ol, 1-amino-2-methylpropan-2-ol, 3-aminopropane-1,2-diol, glucosamine, N-acetylglucosamine, glycine, arginine, lysine, proline, glutamine, histidine, sarcosine, serine, glutamic acid, tryptophan, and mixtures thereof, and the salts such as the potassium, sodium and ammonium salts thereof and mixtures thereof. Especially preferred compounds are glycine, sarcosine, lysine, serine, 2 methoxyethylamine, glucosamine, glutamic acid, morpholine, piperdine, ethylamine, 3 amino-1-propanol and mixtures thereof.

The hair coloring or bleaching compositions may optionally further comprise at least about 0.01% of polymer as an additional thickener, rheology modifiers, stabilizers and/or conditioning agents as described hereinabove in the context of the fiber conditioning treatment composition and any other ingredients commonly utilized in such compositions.

### Method of Use

It is understood that the examples of methods of use and embodiments described herein are for illustrative purposes only and that various modifications or changes in light thereof will be suggested to one skilled in the art without departing from the scope of the present invention.

Another embodiment of the present invention relates to a method of hair treatment, whereby the hair fiber conditioning composition is applied to the hair and worked for a few minutes (to insure uniform application to all of the hair). The composition is then allowed to remain on the hair for a time period of less than about 20 minutes, preferably less than about 15 minutes, more preferably from about 10 seconds to about 10 minutes. The consumer then rinses his/her hair thoroughly with water and allows it to dry and or styles the hair as usual.

According to an alternative embodiment of the present invention, the method of hair treatment is also a sequential hair treatment method comprising the steps of between two and 30 sequential hair fiber conditioning composition treatments wherein the time period between each treatment is from 1 to 30 days, preferably from 1 to 14 days, more preferably from 1 to 7 days, even more preferably from 1 to 5 days and most preferably from 1 to 3 days. In such embodiments, the time that the composition is retained on head may be less than about 15 minutes and is preferably less than about 10 minutes and most preferably from about 10 seconds to about 5 minutes. This method allows the consumer to maintain the treatment benefits over the wash and styling cycle until the next coloring event.

According to the present invention the hair fiber conditioning compositions may be sold individually or may be sold as part of a hair coloring, bleaching kit or highlighting kit such as oxidative hair coloring, bleaching or highlighting kits. These compositions may comprise the fiber conditioning treatment composition as part of the oxidative hair coloring, bleaching or highlighting compositions' container, or may be provided separately in a third container. In the latter case, all three compositions can be mixed immediately before use and applied together, or the content of the third container can be applied (after an optional rinse step) as a post-treatment immediately after the oxidative dye composition or bleaching composition resulting from the mixture of the other containers.

Such oxidative hair coloring compositions are usually sold in kits comprising, in individually packaged components such as separate containers, a coloring or dye component (also called "dye cream" for emulsions or "dye liquid" for solutions) comprising the oxidative colorant or dye, precursors and alkalizing agent which is typically ammonia in a suitable carrier and; a hydrogen peroxide component (also called "hydrogen peroxide cream" for emulsions or "hydrogen peroxide liquid" for solutions) comprising the oxidizing agent (usually hydrogen peroxide). The consumer mixes the coloring component and hydrogen peroxide component together immediately before use and applies it onto the hair. Such kits further comprise an after coloring conditioning composition.

Similarly, bleaching compositions are also usually sold as a kit comprising individually packaged components typically in separate containers. The first component comprises the ammonium ion source (e.g. ammonia), the second component comprises the oxidizing agent and the third (optional) component comprises a second oxidizing agent. The bleaching compositions are obtained by mixing the above-mentioned compositions immediately before use and application to the hair. These kits also typically further comprise an after bleaching conditioning composition.

After working the mixture for a few minutes (to insure uniform application to all of the hair), the oxidative dye or bleach composition is allowed to remain on the hair for an amount sufficient for the dyeing or bleaching to take place (usually from about 2 to 60 minutes, typically about 30 to 45 minutes). The consumer then rinses his/her hair thoroughly with water. The conditioning treatment of the present invention can then be applied and optionally rinsed from the hair.

In another embodiment of the present invention the oxidative hair dye or bleaching compositions may comprise as an optional fourth component a color refresher composition. Such color refresher compositions comprise at least one pre-formed dye and may be applied to the hair immediately after the oxidative color i.e. from about 1 minute after oxidative hair dye or bleach application to 60 days after the application. Such color refresher compositions can be used to increase the initial color obtained and or boost the color during the wash and style cycle until the next oxidative coloring or bleaching event. Alternatively the color refresher composition may be comprised within the fiber or hair fiber conditioning composition thereby ensuring a color boost with each application of the fiber or hair fiber conditioner treatment.

In yet another embodiment of the present invention the hair coloring and or bleaching and or highlighting kits further comprises a device for the application of the composition onto the hair of the consumer. Such devices are known in the art and include, brushes and combs, which may be directly attached to the composition container(s) or used separately, and highlighting caps and foils and the like.

### Test methods:

### Viscosity of functionalized and non functionalised silicone fluids - measurement protocol

An AR 500 rotational rheometer (TA Instruments Ltd., Leatherhead, Surrey KT22 7UQ, UK) is used to determine the viscosity of the functionalized silicone fluids used herein. The determination is performed at 30°C, with the 4cm 2° steel cone measuring system set with a 49µm (micron) gap and is performed via the programmed application of a shear stress of 0.5 to 590 Pa over a 2 minute time period. These data are used to create a shear rate vs. shear stress curve for the material. This flow curve can then be modelled in order to provide a material's viscosity. These results were fitted with the following well-accepted Newtonian model: Viscosity, µ = σ/γ (where σ is shear stress; γ is shear rate)

### Method for assessing silicone particle size within a product

A microscope (Nikon Eclipse E800) is utilised to determine the silicone particle size in the final product. Typically, pictures are taken (JVC color video camera KY-F50) of the final product at a magnification ranging from 100x to 400x. Using the captured image a scale is superimposed (Image software ― Lucia G Version 4.51 (build 028), Laboratory Imaging) previously calibrated using a 100 µm Graticule (Graticules Ltd, Tonbridge Wells, Kent, England) and compared to the average silicone particle within the sample to provide an estimation of particle size.

**Example formulations: Fiber conditioning treatment compositions**

| | #1 | #2 | #3 | #4 |
|---|---|---|---|---|
| Demineralised water | qs to 100 | qs to 100 | qs to 100 | qs to 100 |
| Cetyl alcohol | 2.3 | 2.3 | 4.0 | 4.0 |
| Stearyl alcohol | 2.3 | 2.3 | 2.0 | 2.0 |
| Ceteareth-25 | 1.5 | 1.5 | - | - |
| Phonoxyethanol | 0.1 | 0.1 | - | - |
| Sodium benzoate | 0.1 | 0.1 | - | - |
| Tetrasodium EDTA (87%) | 0.1 | 0.1 | 0.1 | 0.1 |
| Stearamidopropyldimethyamine | - | - | 1.6 | 2.0 |
| L-Glutamic acid | - | - | 0.5 | 0.7 |
| Dicetyldimonium chloride | - | - | 0.5 | |
| Benzyl alcohol | - | - | 0.3 | 0.3 |
| | | | | |
| (Silicone Premix) | (3) | (5) | (5) | (7,5) |
| Example 1 silicone polymer | 3 | - | 2 | - |
| Example 2 silicone polymer | - | 5 | - | 2.5 |
| Dow Coming 200 Fluid 300,000 cst | - | - | 3 | 5 |

## Claims

1. A fiber conditioning treatment composition comprising a functionalised aminosilicone polymer according to the formula: 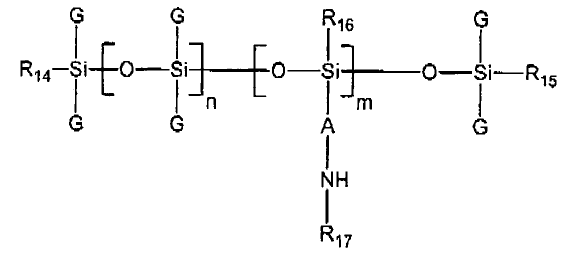 wherein, R₁₄, R₁₅, R₁₆, may be identical or different, and are selected from a hydroxyl radical, C1-C4 alkoxy radicals, methyl and mixtures thereof,
A is selected from linear and branched C3-C8 alkyl radicals and mixtures thereof,
R₁₇ is selected from H, phenyl, linear or branched C1-C4 alkyl radical, benzyl, linear or branched (C1-C8)NH₂ and mixtures thereof,
G is selected from H, phenyl, hydroxyl, C1-C8 alkyl and mixtures thereof,
wherein said functionalised aminosilicone polymer is a random type or block type and, wherein m and n are both at least 1 or greater, the sum of (n+m) is greater than 50, and wherein the average ratio of m:n is from 1:2000 to 1:200.

2. A fiber conditioning treatment composition according to claim 1, wherein said composition further comprises a non functionalised silicone polymer, wherein said non functionalised silicone polymer is alkyl, phenyl or aryl substituted and, wherein the viscosity at 30° of said non functionalised silicone is greater than 60000cps.

3. A method of hair fiber treatment comprising the steps of applying a composition according to any one of the preceding claims to the hair.

4. A method of sequential hair fiber treatments comprising the steps of at least two sequential hair treatments wherein the time period between each treatment is from 1 to 30 days and wherein each treatment comprises the steps of providing a composition comprising a functionalised aminosilicone polymer according to claim 1, to the hair.

5. A method of sequential hair fiber treatments according to claim 4, wherein prior to said sequential hair treatment, said hair is pre-treated with an oxidative coloring or bleaching composition.

6. The use of an aminosilicone functionalised polymer according to claim 1, to condition hair, preferably colored hair.

7. A hair fiber treatment kit comprising
i) an individually packaged oxidizing component
ii) an individually packaged coloring component and
iii) an individually packaged hair treatment composition according to claim 1.

8. A composition according to any one of the claims 1 to 4, wherein in said aminosilicone functionalised polymer G is methyl, R₁₄, R₁₅, R₁₆, which may be identical or different, are selected from a hydroxyl radical, C1-C4 alkoxy radicals and methyl, A is selected from (CH₂)₃ or (CH₂CHCH₃CH₂) and R₁₇ is selected from H or (CH₂)₂NH₂, and wherein the average ratio m:n is 1:1500 to 1:200, and the sum of m+n is in the range 50 to 2000.

9. A composition according to any one of the claims 1 to 4, wherein said composition comprises from 0.1 % to 10 % by weight of said functionalised aminosilicone polymer.

10. A composition according to claim 1, wherein said functionalised aminosilicone has a particle size of greater than 2 microns.

11. A composition according to claim 2, wherein said composition comprises from 0.1 % to 9%, preferably 0.2 % to 8 %, most preferably 0.5 % to 7 %, by weight of said non functionalised silicone polymer.

12. A composition according to claims 1 and 2, wherein the weight ratio of said functionalised aminosilicone polymer to said non functionalised silicone polymer, is from 5 : 95 to 95 : 5.

13. A composition according to any one of the preceding claims, wherein said composition further comprises an adjunct selected from thickeners and additional conditioning agents.

14. A composition according to any one of the preceding claims, wherein said composition further comprises at least one preformed hair coloring agent.
